# EUROPEAN PATENT APPLICATION

(11) **EP 4 023 146 A1**
(43) Date of publication of application: **06.07.2022**
(21) Application number: 21216117.8
(22) Date of filing: 20.12.2021
(51) Int. Cl.: A61B 5/00, A61B 5/11

(54) **CONTACTLESS PATIENT MONITORING SYSTEM WITH AUTOMATIC IMAGE IMPROVEMENT**

(30) Priority: 30.12.2020 US 202063132004 P
(71) Applicant: Hill-Rom Services, Inc., Batesville, IN 47006-9167 (US)
(72) Inventor: SOREEFAN, Ibne, Batesville, 47006-9167 (US); HOLMES, Tyler, Batesville, 47006-9167 (US); LANE, John, Batesville, 47006-9167 (US); AGDEPPA, Eric D., Batesville, 47006-9167 (US)
(74) Representative: Findlay, Alice Rosemary

(57) **Abstract**

A patient monitoring system comprises a contactless monitoring unit having a contactless patient monitor and operable to communicate with equipment in a patient room and components of hospital information system. The patient monitoring system implements methods for detecting and optimizing signals from a patient supported on a patient support apparatus. The patient monitoring unit includes a power source and a controller. The contactless patient monitor includes a first detector, an emitter, and a controller. The controller processes signals from the first detector and vary the operation of the emitter to change the environment in the field of the detector such that the signals detected by the first detector are optimized.

## Description

The present disclosure relates to tracking and monitoring systems and methods for monitoring patients in a clinical setting. More specifically, the system and method relates to using multiple imaging devices to monitor patient movement and vital signs in varying environments.

Patients in a care facility such as a hospital, for example, require varying degrees of interaction with caregivers. Some patients may have conditions that need constant monitoring of their vital information. Certain protocols may be enforced in response to changes in a patient's vital information. Thus, real time monitoring of a patient's vital information may provide caregivers with critical information to treat and respond to patients immediately.

Monitoring of positioning when the patient is in a patient support apparatus, such as a hospital bed, in real time increases the quality of the patient's care as changes in patient condition is detected and caregivers can be alerted to potential risks. For example, a patient may be at a risk of falling and injuring themselves if they exit the patient support apparatus and monitoring of their movement relative to the patient support apparatus provides feedback for determining if they are attempting to exit from the patient support apparatus. Regular monitoring of respiration rate, heart rate, temperature, and the like, are beneficial in monitoring the patient's progression of care. A problem with using imaging techniques to monitor patients is the variability of the patient environment. Differences in lighting including changes due to ambient light, as well as variability between patient rooms, makes it difficult to use a standard approach for imaging. Also, the need to lower lighting while the patient is resting may further exacerbate the performance of image monitoring systems for gathering patient vital signs from images. Because of these issues, there is an ongoing need to improve the accuracy and reliability of these measures to maximize the benefit to the patient under care.

A method, system or apparatus may comprise one or more of the following features, alone or in any combination:
According to a first aspect of the present disclosure, a patient monitoring system comprises a contactless monitoring unit having a contactless patient monitor and operable to communicate with equipment in a patient room and components of a hospital information system. The patient monitoring unit also includes a power source and a controller.

In some embodiments, the contactless patient monitor includes a first detector, an emitter, and a controller. The controller may be operable to process signals from the first detector and vary the operation of the emitter to change the environment in the field of the detector such that the signals detected by the first detector are optimized.

In some embodiments, the detector is a monochromatic camera. The monochromatic camera may be a high-resolution monochromatic camera.

In some embodiments, the operation of the emitter may be varied by the controller of the contactless patient monitor by changing the position of the emitter. In some embodiments, the operation of the emitter may be varied by varying the wavelength emitted by the emitter. In some embodiments, the operation of the emitter may be varied by varying the intensity of the emitter.

In some embodiments, the contactless patient monitor may include more than one emitter and the operation of the each of the emitters may be varied to optimize the signals detected by the first detector. In some embodiments, the operation of one or more of the emitters may be varied by the controller of the contactless patient monitor by changing the position of the emitter. In some embodiments, the operation of one or more of the emitters may be varied by varying the wavelength emitted by the emitter. In some embodiments, the operation of one or more of the emitters may be varied by varying the intensity of the emitter.

According to a second aspect of the present disclosure, a contactless patient monitor comprises a controller, at least one emitter, a first detector, and a second detector, the controller controlling the monitor. The controller includes a processor and a non-transitory memory device that includes instructions that are performed by the processor to control the monitor. The controller varies the operation of the emitter in response to the environmental conditions in its field of view as detected by the first detector to improve the image captured from the field of view by the first detector.

In some embodiments, the first detector is a high-resolution monochromatic detector operable to detect in its spectrum. The second detector is a low-resolution thermal camera.

In some embodiments, the emitter has a variable output. In some embodiments, the operation of the emitter is varied by altering its wavelength. In some embodiments, the operation of the emitter is varied by altering its intensity. In some embodiments, the operation of the emitter is varied by altering its position to reduce shadows in the field of view.

In some embodiments, there are a plurality of emitters. In some embodiments, the operation of or more of the plurality of emitters is varied by altering its wavelength.

In some embodiments, the operation of or more of the plurality of emitters is varied by altering its intensity. In some embodiments, the operation of or more of the plurality of emitters is varied by altering its position to reduce shadows in the field of view.

According to a third aspect of the present disclosure, a method of monitoring a patient includes controlling the operation of at least one emitter, and at least one detector, the method comprising varying the operation of the emitter in response to the environmental conditions in its field of view as detected by the at least one detector to improve an image captured by the at least one detector.

In some embodiments, the detector is a high-resolution camera and the operation of the emitter is varied based on the camera signal.

In some embodiments, varying the operation of the emitter includes altering its wavelength.

In some embodiments, varying the operation of the emitter includes altering its intensity.

In some embodiments, varying the operation of the emitter includes altering its position to reduce shadows in the field of view.

In some embodiments, a plurality of emitters are positioned to emit into a field of view of the detector and the operation of multiple ones of the plurality of emitters is varied based on the camera signal.

In some embodiments, varying the operation of at least one emitter includes altering its wavelength.

In some embodiments, varying the operation of at least one emitter includes altering its intensity.

In some embodiments, varying the operation of at least one emitter includes altering its position to reduce shadows in the field of view.

According to a fourth aspect of the present disclosure, a contactless monitoring unit for monitoring a patient in a care environment comprises a contactless monitor including at least one emitter and first and second detectors, and a controller. The controller includes a processor and a non-transitory memory device. The non-transitory memory device includes instructions that, when executed by the processor, cause the contactless monitoring unit to acquire information from the environment in which a patient being monitored by the contactless monitoring unit is located and modify operation of the contactless monitor based on the information from the environment.

In some embodiments, the first detector is a high-resolution monochromatic detector and the second detector is a low-resolution infrared camera. The controller varies the operation of the emitter in the contactless monitoring sensor in response to the environmental condition in the field of view of the contactless monitoring sensor as detected by the monochromatic detector in the contactless monitoring sensor.

In some embodiments, the contactless monitoring unit is in communication with a patient support apparatus and is operable to receive information from the patient support apparatus to adjust the operation of the contactless monitoring unit or changes operation of the patient support apparatus to improve the quality of image captured by the detectors. The field of view as detected by the monochromatic detector in the contactless monitoring sensor includes a patient on the support apparatus. The field of view as detected by the infrared detector in the contactless monitoring sensor includes a patient on the support apparatus and overlaps the field of view of the monochromatic detector.

In some embodiments, the contactless monitoring unit is in further communication with an in room computer to gather additional information from other sensors in the patient room to adjust the operation of the contactless monitoring unit.

In some embodiments, the controller defines an operating boundary for the contactless monitor by processing the input from the field of view of the monochromatic detector.

In some embodiments, the controller identifies at least one region of interest as a contour by processing signals from both detectors in tandem.

In some embodiments, a first region of interest includes the patient's face, wherein the centroid of the patient's face is located on a thermal image obtained from the second detector and mapped onto a grayscale image obtained from the first detector using geometric transformation.

In some embodiments, any changes in the thermal image are updated on the grayscale image, altering the region of interest in real time.

In some embodiments, the controller obtains vital information about the patient's health by processing the pixel values obtained from the region of interest in the grayscale image.

In some embodiments, the controller obtains raw data from the second detector and convert it into a pseudo colored thermal image by normalizing it and applying a color-map.

In some embodiments, the controller identifies contours in upper and lower zones of the patient support apparatus.

In some embodiments, the controller chooses a contour based on hard coded criteria and contour location.

In some embodiments, the coordinates of the chosen contour is transformed into the coordinates obtained from the monochromatic detector by scaling it by a factor of the monochromatic detector's resolution divided by the infrared detector's resolution and shifting each point by X and Y offsets determined by the common pixels within their intersecting fields of view of both detectors.

In some embodiments, the controller obtains vital information about the patient's health by processing the pixel values obtained from the contours in the grayscale image.

The invention will now be further described by way of example with reference to the accompanying drawings, in which:
Fig. 1 is a diagrammatic view and partial perspective view of a patient monitoring system in accordance with the present disclosure showing the contactless monitoring unit cooperates with a patient support apparatus positioned in a room;
Fig. 2 is a diagram representing the different components of the patient monitoring system and their respective communication paths;
Fig. 3 is a perspective view of a contactless monitoring unit mounted to the ceiling of hospital room, the contactless monitoring unit including multiple detectors with overlapping detection fields;
Fig. 4 is a block diagram representing the different components of the contactless monitoring unit in communication with the patient support apparatus;
Fig. 5 is a flow chart representing the various steps involved in contactless patient monitoring that results in the acquisition of vital signs information of the patient;
Fig. 6 is a diagrammatic representation of the data detected by a first detector overlaid upon the environment in the detection field to show the data collected by the first detector;
Fig. 7 is a diagrammatic representation of the data detected by a second detector overlaid upon the environment in the detection field to show the data collected by the second detector;
Fig. 8 is a flow chart representing the steps involved in determining the operation of an emitter in the contactless monitor;
Fig. 9 is a diagrammatic representation, similar to Fig. 6, of the data detected by the first detector overlaid upon the environment in the detection field to show the data collected by the first detector, the patient in Fig. 9 in a different position than that shown in Fig. 6;
Fig. 10 is a diagrammatic representation, similar to Fig. 7, of the data detected by the first detector overlaid upon the environment in the detection field to show the data collected by the first detector, the patient in Fig. 10 in a different position than that shown in Fig. 7; and
Fig. 11 is a flow chart representing the detailed process of image acquisition by two detectors in the contactless monitoring unit operating in tandem.

In the following description, numerous specific details such as logic implementations, types and interrelationships of system components, and logic partitioning/integration choices are set forth in order to provide a more thorough understanding of the present disclosure. One skilled in the art, however, appreciates that the disclosure may be practiced without such specific details. In other instances, control structures, gate level circuits, and full instruction sequences have not been shown in detail.

Embodiments of the disclosure may be implemented in hardware, firmware, software, or any combination thereof. Embodiments of the disclosure may also be implemented as instructions stored on a machine-readable medium, which may be read and executed by one or more processors. A machine-readable medium may include any mechanism for storing or transmitting information in a form readable by a machine (e.g., a computing device). For example, a machine-readable medium may include read only memory (ROM); random access memory (RAM); magnetic disk storage media; optical storage media; flash memory devices; and others.

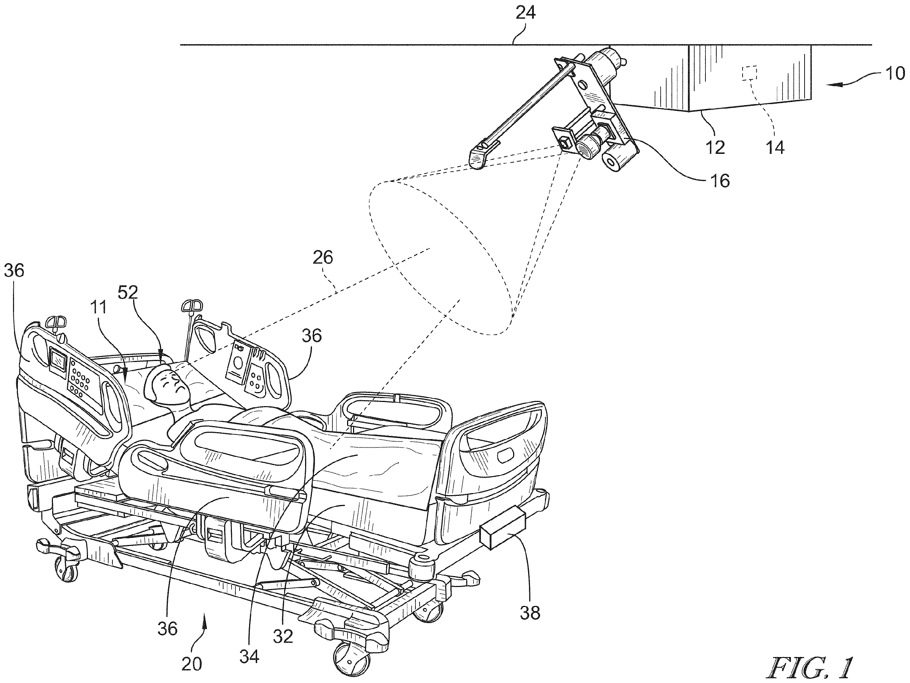

A first embodiment of a contactless patient monitoring system 10 in accordance with the present disclosure is shown in Fig. 1. It includes a contactless monitoring unit 12 configured to execute a contactless patient monitoring process as will be described in further detail below. The contactless monitoring unit 12 includes a contactless monitor 16, and a controller 14. The contactless patient monitoring system 10 receives data from one or more sources, determines regions of interest for monitoring a patient's vital information, and monitors the regions identified. The present disclosure is directed to using components of the contactless monitor 16 to improve the accuracy of the data gathered by the contactless monitor 16, including the use of an emitter 306 of the contactless monitor 16 to emit signals that optimize the waves reflected from the environment to improve the signals detected by the contactless monitor 16.

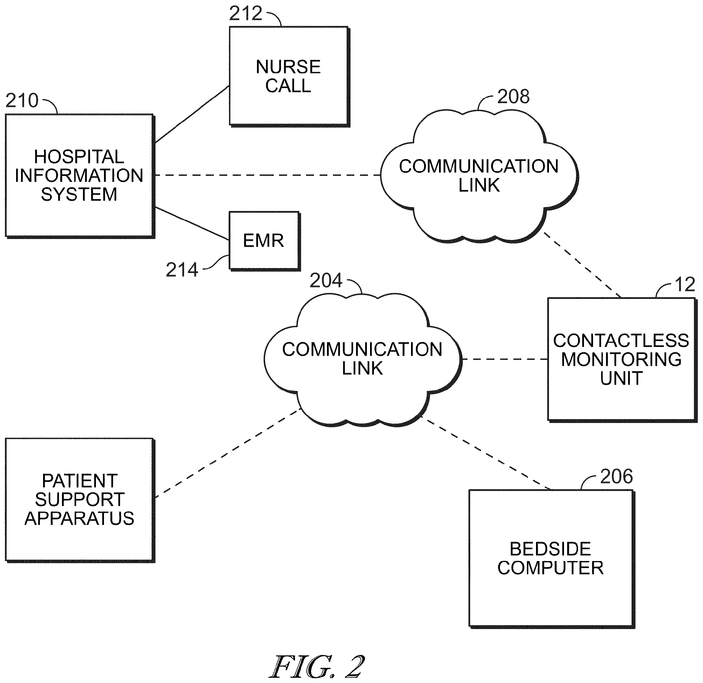

Referring to Fig. 2, the controller 14 of the contactless monitoring unit 12 is further coupled to a communication link 208. The communication link 208 is configured to provide communication between the controller 14 and a hospital information system 210. The hospital information system 210 is coupled to, for example, a centralized nurse call system 212 and a centralized electronic medical record system 214. Both the nurse call system 212 and electronic medical records system 214 include information that is related to the patient support apparatus 20 and associated with the patient 11 stored in memory as related records. The information related to the patient 11 stored in memory in the nurse call system 212 and electronic medical records system 214 is constantly updated as information is added to the electronic medical records system by caregivers and the nurse call system 212 receives information related to the patient 11, the patient support apparatus 20, and the contactless patient monitoring system 10.

In some embodiments, the communication link 208 may involve a communication infrastructure such as an Ethernet of a healthcare facility in which the contactless monitoring unit 12 is located. It is within the scope of this disclosure for the communication link 208 to enable communication with other computers that are part of the hospital information system 210 such as those included as part of a nurse call system, a physician ordering system, an admission/discharge/transfer (ADT) system, or some other system used in a healthcare facility in other embodiments. In some embodiments, the communication link 208 may facilitate bidirectional communication with various infrastructure that, in turn, communicate bidirectionally with other devices and systems. It may be a wired communication link in some embodiments and a wireless communications link in other embodiments. Thus, communication link 208, in some embodiments, comprises a cable that connects the contactless monitoring unit 12 to a wall mounted jack or a network interface.

The patient 11, as shown in Fig. 1, is supported on the patient support apparatus 20. The patient support apparatus 20 includes a support frame 32, a patient support surface 34, and one or more side rails 36. The support frame 32, the patient support surface 34, and the side rails 36 cooperate to maximize patient comfort and patient safety. The patient support surface 34 is positioned on the support frame 32 and located between the patient 11 and the support frame 32 to maximize patient comfort and minimize skin damage of the patient 11. The side rails 36 are coupled to the support frame 32 to move between lowered positions and raised positions as shown in Fig. 1. The lowered positions are associated with providing patient care or patient ingress and egress. The raised positions are associated with blocking patient ingress and egress.

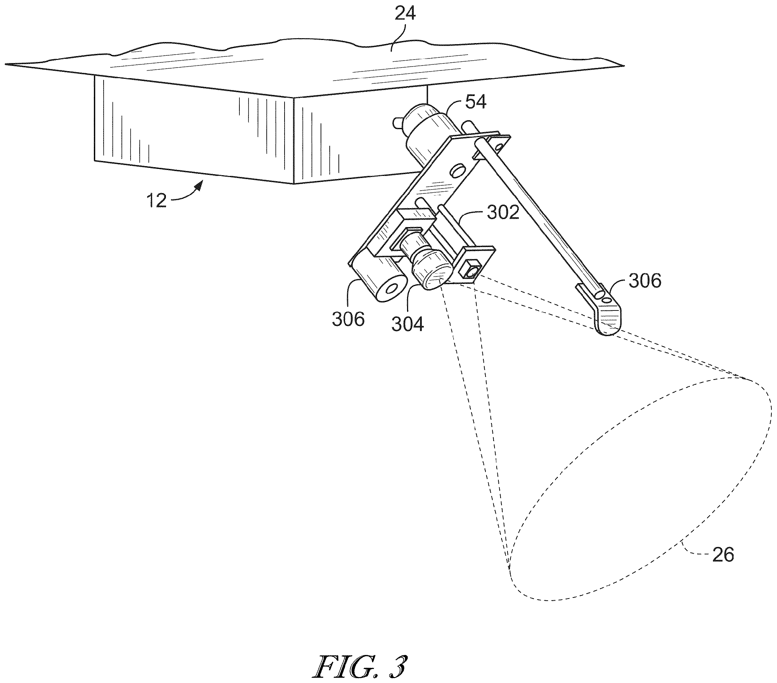

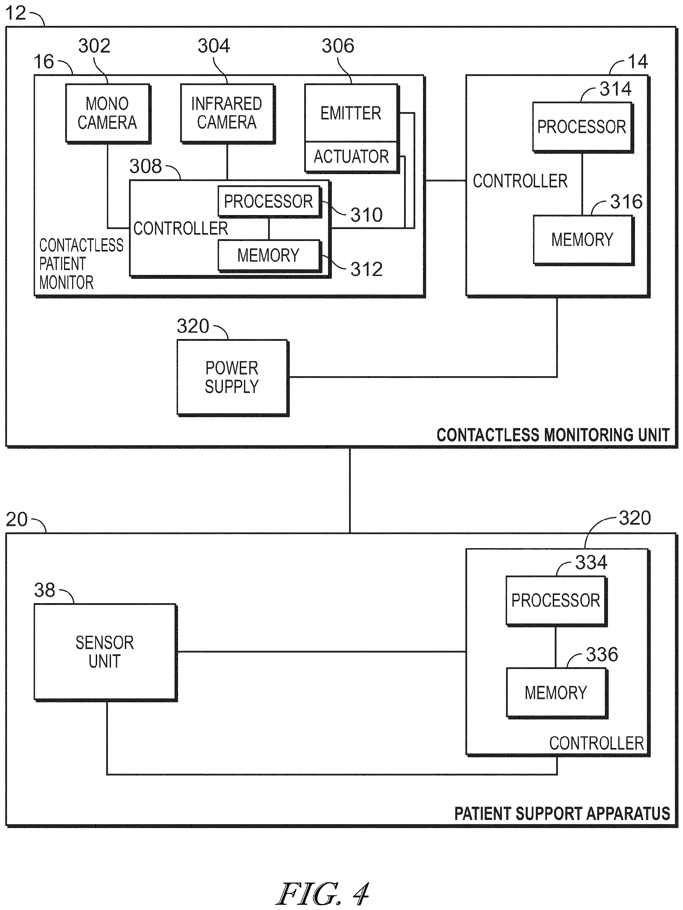

Referring to Fig. 4, the contactless monitoring unit 12 includes a power source 320 such as a power converter for converting power from a mains power source or a battery, for example, the contactless monitor 16, and a controller 14. In the illustrative example, the contactless monitor 16 is coupled to a ceiling 24 in the patient room in a spaced-apart relation to the patient 11, as shown in Fig. 1 and 3. The contactless monitor 16 may communicate with the controller 14 via a wired connection or a wireless connection. The contactless monitor 16 may have a number of detectors and emitters. In an embodiment, as illustrated in Fig. 3, the contactless monitor 16 includes a detector embodied as a monochromatic camera 302 configured to detect electromagnetic radiation in the appropriate spectrum and output a grayscale image, another detector embodied as an infrared camera 304 configured to detect the signature of an object in accordance with its thermal heat pattern and output a thermal image, and at least one emitter 306. In one embodiment, the contactless monitor 16 includes only the monochromatic camera 302. In another embodiment, the contactless monitor 16 includes only an infrared camera 304. In still yet another embodiment, the contactless monitor 16 includes both a monochromatic camera 302 and an infrared camera 304, but no emitter 306. As will be described in further detail below, the emitter 306 emit spectral radiation of a particular wavelength which is then coordinated with one or other of the cameras 302 or 304 to improve the image detected by the respective camera 302, 304. For example, the emitter 306 may be adjusted to vary a wavelength emitted to improve the reflection from an area around a patient and thereby improve the image captured by one or the other of the respective cameras 302, 304. Also, the controller 308 may vary the intensity of the emitter 306. The emitter 306 is supported from an actuator 52 that is operable to vary the position of the emitter 306 to reduce shadows in the field of view of one or the other of the cameras 302, 304. Still further, the contactless monitoring unit 12 may include multiple emitters 306 positioned throughout the room, each of the emitters 306 being controllable by the contactless monitoring unit 12 to vary the operation of each emitter 306.

The contactless monitor 16 is positioned to lie in a spaced-apart relation to the patient 11 to detect electromagnetic radiation from a field of view 26 in which the patient 11 is located. The contactless monitor 16 includes a controller 308 that evaluates the field of view 26 and alters the operation of the emitter(s) 306 and/or detector(s) 302, 304 in real time. The controller 308 in the contactless monitor 16 may then convert the detected electromagnetic radiation into a signal that is indicative of patient data 18. The sensor signal of patient 11 is then communicated to the controller 14 for processing to determine regions of interest for monitoring a patient's vital information. In some embodiments, the controller 14 may detect and/or convert the detected electromagnetic radiation into a signal before processing it to determine regions of interest for monitoring a patient's vital information. The controller 14 may communicate with the contactless monitor 16 via a wired or wireless connection.

The contactless monitoring unit 12 is further coupled to a communication link 204 as shown in Fig. 2. The communication link 204 is configured to provide communication between the controller 14 and patient support apparatus 20, and between the controller 14 and a bedside computer 206. In some embodiments, communication link 204 comprises wireless signals sent between the patient support apparatus 20 and a wireless interface. Communication link 204 may comprise one or more wired links and/or wireless links as well, according to this disclosure.

In one embodiment, the controller 14 is directly coupled to the patient support apparatus 20 through the communication link 204 and signals the patient support apparatus 20 to perform one or more predetermined actions in response to receiving and processing the signals. In another embodiment, the controller 14 is coupled to contactless monitor 16 in a spaced-apart relation to the patient 11 on patient support apparatus 20. The controller 14 may also communicate with the contactless monitor 16, via controller 308, any change in the position of the patient 11. In some embodiments, the controller 14 may perform a predetermined series of evaluations 22 as suggested in Fig. 1. The predetermined evaluation 22 is, for example, to monitor operation boundary, identify regions of interest, and calculate vital information of the patient 11. The information gathered may be used for determining patient need as disclosed in U.S. Patent Application Publication No. 2015/0294549A1, or monitor patient movement as disclosed in U.S. Patent No. 9,301,689, each of which is incorporated in their entirety for their disclosure of analysis of imaging data.

In one embodiment, as illustrated in Fig. 1, a bed sensor unit 38 is coupled to the patient support apparatus 20 and configured to provide bed data to the controller 14 of the contactless monitoring unit 12. Bed data may include, for example, an arrangement of the support frame 32, a status of the patient support surface 34, and the positions of the side rails 36. The controller 14 receives the bed sensor signal and uses the bed data 40 to identify patient position, among other things. The controller 14 may also use the bed data 40 to determine factors associated with determining the vital information of the patient 11, or to identify an unfavorable position for patient 11. The controller 14 of the contactless monitoring unit 12 may also receive data from the hospital information system 210 that may affect the processing of vital information.

In one embodiment, as illustrated in Fig. 4, the patient support apparatus 20 may have a sensor 44 that may signal the presence or position of the patient, and a controller 330. The presence or absence of a patient may cause the modification of the operation of the contactless monitoring unit 12. The bed sensor unit 38 may receive data from one or more sources such as other parts of the patient support apparatus, and determine regions of interest for monitoring a patient's vital information based on position. Controller 330 is in communication with controller 14. In some embodiments, controller 330 may be in communication with controller 308.

The controller 14 associated with the patient support apparatus 20 includes inputs that provide information to the processor 314. Processor 314 is coupled to a non-transitory memory device 316 which includes instructions that, when performed by the processor 314, cause the processor to perform the algorithms described herein. The inputs may include the bed sensor unit 38 or other sensors 44 included in the patient support apparatus 20. These sensors may include, but are not limited to frame position sensors, siderail position sensors, support surface sensors, a scale system, and caster brake sensors. The frame position sensors provide information regarding the position of various components of the patient support apparatus 20. Information provided may include the height of the patient support apparatus, the inclination of a head section of the support frame 32, the degree of tilt of an upper frame included in the support frame, or any other frame position data that might be available from frame position sensors of the particular patient support apparatus 20.

The side rail position sensors provide an indication to the controller 14 of whether a particular side rail 36 of the patient support apparatus 20 is in a raised or lowered position, which may be used to adjust the processing of the images. It is contemplated that additional sensors may be implemented which indicate whether a particular side rail is latched into a particular position. The support surface sensors provide information regarding the operation of the patient support surface 34, such as an inflatable/pneumatic mattress, of the patient support apparatus 20. Such a patient support surface 34 may be integrated into the support frame 32 of the patient support apparatus 20 or may be a separate structure that is operated generally independently of the patient support apparatus 20, but communicates with the controller 14 of the patient support apparatus 20.

The support surface sensors may include pressure sensors that identify pressures in particular inflatable structures of the support surface or they may include position sensors. For example, accelerometers positioned in particular locations within the patient support surface 34 may provide feedback regarding the amount of inclination of a particular section of the patient support surface 34 relative to gravity, independent of the frame position sensors. The support surface sensors may also provide information regarding the degree of lateral rotation of a patient supported on the support surface. In addition, the patient support apparatus 20 may include a support surface pressure control system that controls the pressure in one or more air bladders in the patient support surface 34. Information from the support surface sensors may be used to determine patient position and its impact on vital information calculations.

The scale system provides information to the controller 14 regarding the weight of the patient supported on the patient support apparatus 20. The scale system also provides information regarding the position of a patient on the patient support apparatus 20 and may provide information regarding the degree of movement of the patient. The data from the scale system may also be used by the controller 14 to determine if an unexpected weight has been added to the patient support apparatus 20 or provide other data regarding activities around the patient support apparatus 20. For example, the support surface sensors and scale system may cooperate to determine that a patient is sitting up on a patient support apparatus 20 without having the head section raised. Information from the scale system may be used to determine patient position and its impact on vital information calculations. In some embodiments, controller 14 may communicate the information gleaned from the above sensors to controller 308.

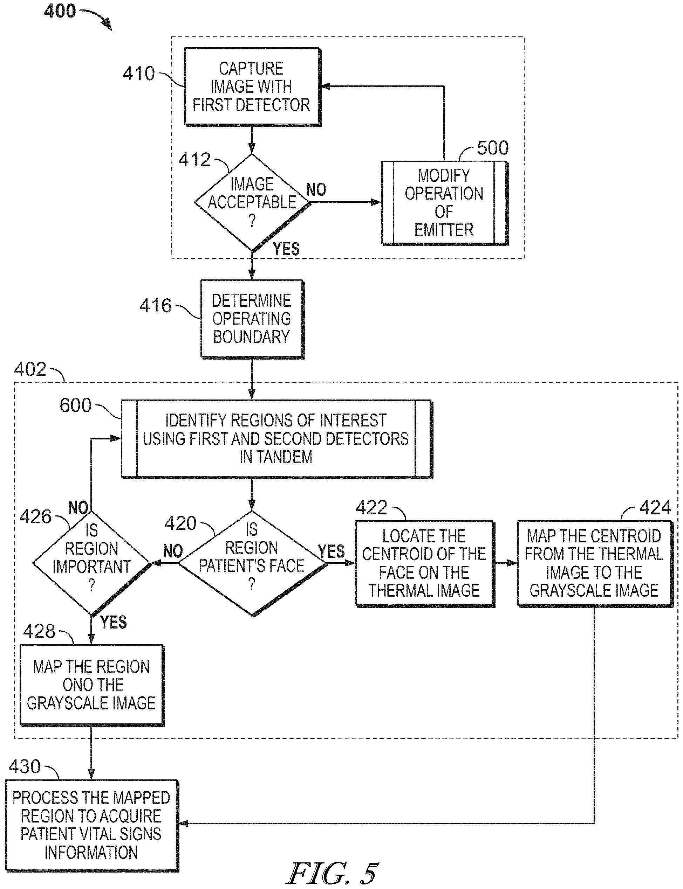

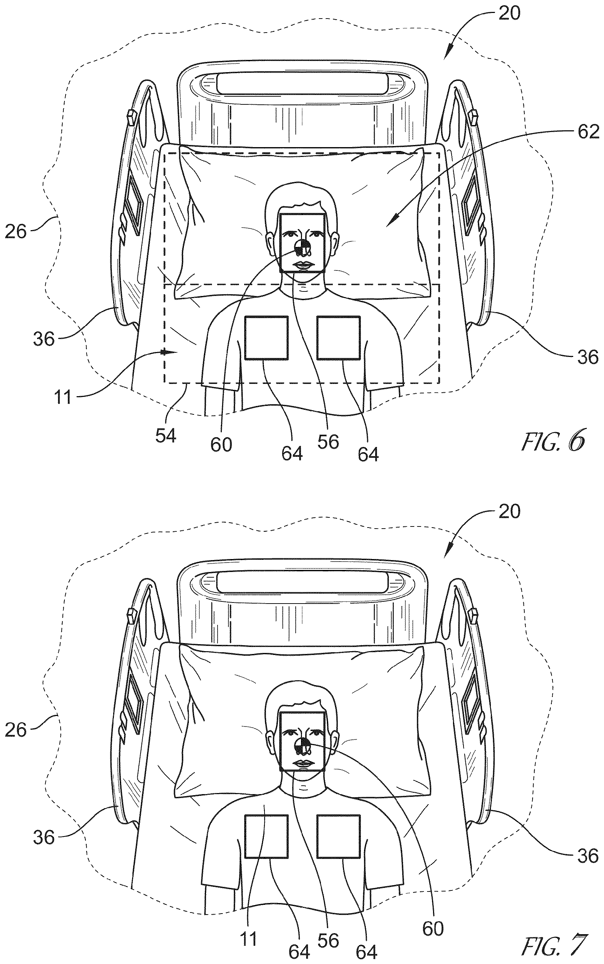

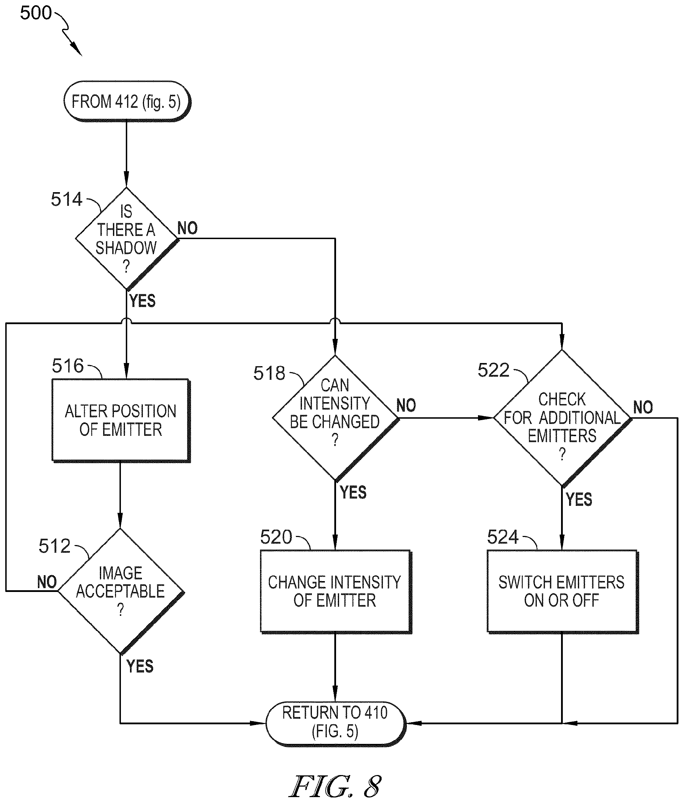

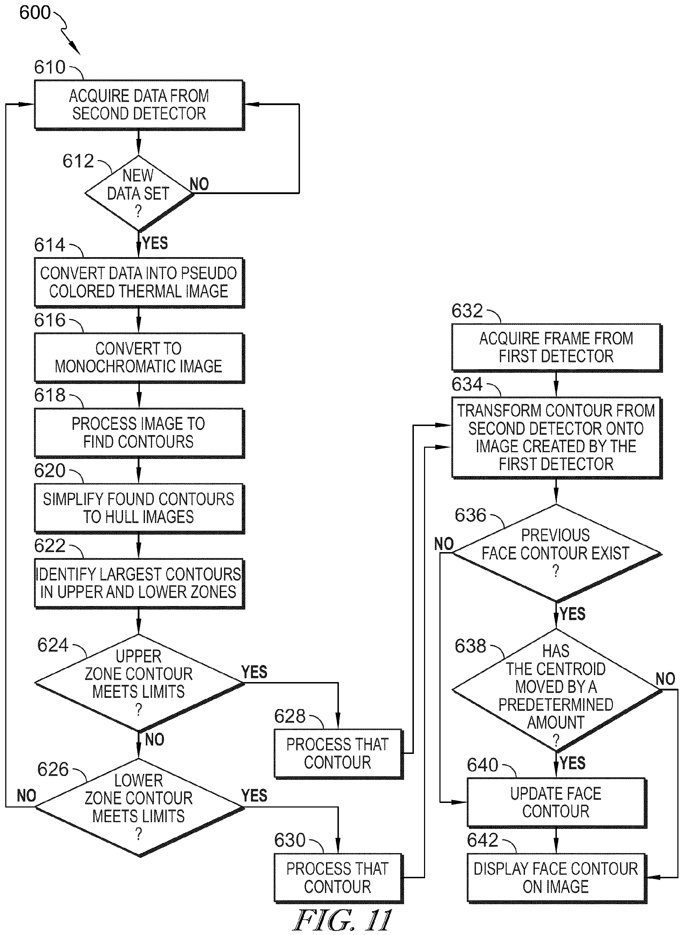

The various steps involved in contactless monitoring that result in the acquisition of vital signs information of the patient are as illustrated in the flowchart 400 and shown in Fig. 5. At step 410, the controller 308 in the contactless monitor 16 captures the field of view 26 with the monochromatic camera 302. In step 412, the controller 308 evaluates if the environment captured in field of view 26 is acceptable for the acquisition of vital information. If it is not, the controller 308 alters the operation of the emitter(s) 306 in the contactless monitor 16 in sub-process 500 and as further detailed in Fig. 8. If the environment captured in field of view 26 is acceptable for the acquisition of vital information, a processor 310 processes the pixels captured by the monochromatic camera 302 to determine an operating boundary 54 at step 416 and shown in Fig. 6. The processor 310 is coupled to a non-transitory memory device 312 that includes instructions that are processed by the processor 310 to perform the functions of the processor 310 described herein. In sub-process 600, the controller 308 identifies at least one region of interest 56 by using both detectors/cameras 302, 304 in tandem as explained below and shown in Fig. 11.

Referring again now to Fig. 5, the detectors include one high-resolution monochromatic camera 302 and one low-resolution infrared camera 304. In step 420, the controller evaluates if the region of interest 56 is the patient's face 62. If it is the face, the controller 308 locates the centroid 60 of the patient's face 62 on the thermal image at step 422 as illustrated in Fig. 7. The controller 308 maps the centroid 60 onto a grayscale image, as shown in Fig. 6, using geometric transformation at step 424. The process then proceeds to process the pixel values obtained from the region of interest in the grayscale image to determine patient's vital information at step 430. If the region of interest is not the face 62, the controller 308 decides if the region of interest 64 important for vital information at step 326. If it is, the controller 308, maps it onto a grayscale image using geometric transformation at step 328, and then proceeds to process the pixel values obtained from the region of interest in the grayscale image to determine patient's vital information at step 430. Alternatively, if the region of interest in not important for vital information, the controller 308 repeat sub-process 600. The overall process of image acquisition is described by the steps enclosed in the block 402, and is further detailed in Fig. 11.

The steps involved in determining the optimal environment of the field of view 26 are as illustrated in the sub-process 500 shown in Fig. 8. The process commences from step 412 and progresses to decision step 514 where the controller 308 evaluates the image captured at 410 to determine if there is a shadow in the field of view 26. If the controller 308 determines there is a shadow, the controller 308 alters the position of the emitter 306 and reevaluates the image at decision step 526. If the image is acceptable at step 526, the controller 308 returns to step 410. If the image is not acceptable at step 526, then the controller 308 progresses to decision step 522 that is described in further detail below.

If the controller 308 determines that there is no shadow at decision step 514, the controller 308 determines if the intensity of the emitter 306 can be increased or decreased at decision step 518 to improve the image. If the intensity of the emitter 306 can be altered, the controller 308 alters the intensity of the emitter 306 at step 520 and returns to step 510. If the controller determines that the intensity of the emitter 306 cannot be altered at decision step 518, the controller 308 determines if there is more than one emitter 306 in the contactless monitor 16 at decision step 522. In embodiments where there is more than one emitter 306, the controller 308 advances to step 524 to switch the one or more emitters 306 on or off to attempt to obtain an optimal field of view 26 and returns to step 510. If there is only one emitter 306, the controller 308 returns to step 510 without any further changes as the environment of field of view 26 captured is the best possible one. In such a case, the controller 308 continues to loop between steps 410, 412 and sub-process 500 until the environment changes such that the image is acceptable at step 412 or modifications to the operation of the contactless monitor 16 result in an acceptable image.

Once an acceptable image is identified, the controller 308 advances to sub-process 600 where full image acquisition is completed. The sub-process 600 includes several process steps illustrated in the sub-process 600 shown in Fig. 11.

First, the controller 308 acquires raw data from the infrared camera 304 at step 610, and acquires the frame from the monochromatic camera 302, at step 632. At decision step 612, the controller 308 determines if the raw data acquired at step 610 is a new data set. If it is not a new data set, then the controller 308 returns to step 610.

If it is determined to be a new data set, the controller 308 converts the raw data into a pseudo colored thermal image by normalizing it and applying a color map at step 614. Then the controller 308 creates a new image where pixels in the previous image with corresponding raw data values that are closest to the maximum raw data value are white, and all other pixels are black at step 616. The controller 308 processes the resulting data to detect contours on the resulting image at step 618. The controller 308 then simplifies the contours into convex hull shapes at step 620. The controller 308 processes the convex hull shapes to find the largest contours in the upper and lower bed zones at step 622. At decision step 624, the controller 308 determines if there is contour in the upper bed zone that fits predetermined hard-coded maximum and minimum size criteria. If such a contour is detected at decision step 624, the controller 308 progresses to step 628 the particular detected contour is chosen for further processing.

If there is no such contour found at decision step 624 the controller 308 determines if there is contour in the lower bed zone that fits the hard-coded maximum and minimum size criteria at decision step 626. If no contours fit the criteria at step 624 or at step 626, the controller 308 returns to step 610. If such a contour is detected, the controller 308 executes step 630, where the particular contour that has been detected is chosen for further processing.

The controller 308 progresses to step 634 where the controller 308 transforms the contour from the coordinates identified in the thermal image obtained from the infrared camera 304 onto the coordinates of the grayscale image obtained from the monochromatic camera 302. The transform is accomplished by scaling the chosen contour by the ratio of the monochromatic camera's resolution divided by the infrared camera's resolution and shifting each point by Cartesian X and Y offsets that are determined by the cameras' common pixels within their intersecting fields of view.

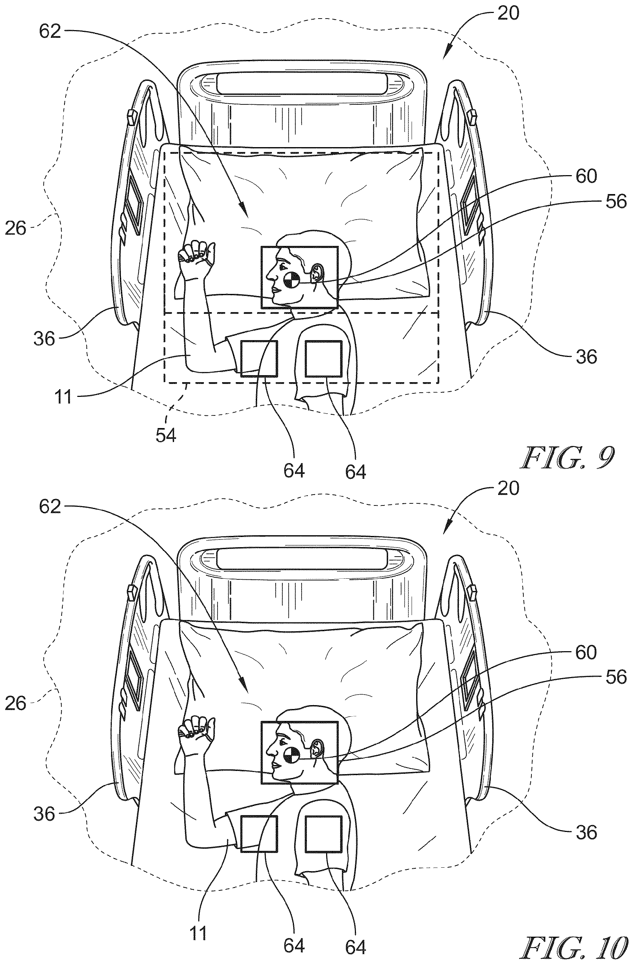

The controller 308 processes the transformed data and compares the transformed data to existing data to determine if a previously transformed face contour exists in the coordinates on the grayscale image obtained from the monochromatic camera 302 at decision step 636. If it does, the controller 308 progresses to decision step 638 and determines if the center coordinate of the face contour on the thermal image has been moved by a predetermined amount of pixels. If the center coordinate of the face contour has been moved by the predetermined amount of pixels, such as that illustrated in Figs. 9 and 10, the controller 308 then updates the face contour at step 640. The controller 308 then progresses to step 642 where the face contour is displayed on the image taken by the monochromatic camera 302.

If the controller 308 determines that a previously transformed face contour does not exist in the coordinates on the grayscale image obtained from the monochromatic camera 302 at step 636, then the step 640 is executed and the face contour is displayed on the image taken by the monochromatic camera 302.

If it is determined at decision step 636 that a previously transformed face contour does exist on the coordinates in the grayscale image obtained from the monochromatic camera 302 as determined at step 636, but the center coordinate of the face contour on the thermal image has not been moved by a predetermined amount of pixels as determined at step 638, the controller 308 does not update the face contour at step 640, and instead executes step 642, wherein the already existing face contour is displayed on the grayscale image taken by the monochromatic camera 302.

The processors described herein may be embodied as any type of processor capable of performing the functions described herein. They may be embodied as a single or multi-core processor(s), digital signal processor, microcontroller, or other processor or processing/controlling circuit. The memory devices, unless otherwise described, may be embodied as any type of volatile or non-volatile memory or data storage capable of performing the functions described herein. In operation, the memory devices may store various data and software used during operation of the systems described herein, such as operating systems, applications, programs, libraries, and drivers.

Although certain illustrative embodiments have been described in detail above, variations and modifications exist.

Embodiments of the invention can be described with reference to the following numbered clauses, with preferred features laid out in the dependent clauses:
1. A contactless patient monitor comprising:
   at least one emitter,
   a first detector, and
   a second detector,
   a controller controlling the monitor, the controller including a processor and a non-transitory memory device that includes instructions that are performed by the processor to control the monitor, and
   wherein the controller varies the operation of the emitter in response to the environmental conditions in its field of view as detected by the first detector to improve the image captured by the first detector from the field of view.
2. The contactless patient monitor of clause 1, wherein the first detector is a high-resolution monochromatic detector operable to detect in its spectrum, and wherein the second detector is a low-resolution thermal camera.
3. The contactless patient monitor of either clause 1 or clause 2, wherein the emitter has a variable output.
4. The contactless patient monitor of clause 3, wherein the operation of the emitter is varied by altering its wavelength.
5. The contactless patient monitor of clause 3 or clause 4, wherein the operation of the emitter is varied by altering its intensity.
6. The contactless patient monitor of any one of clauses 3 to 6, wherein the operation of the emitter is varied by altering its position to reduce shadows in the field of view.
7. The contactless patient monitor of clause 3, wherein there are a plurality of emitters.
8. The contactless patient monitor of clause 7, wherein the operation of one or more of the plurality of emitters is varied by altering its wavelength.
9. The contactless patient monitor of either clause 7 or clause 8, wherein the operation of or more of the plurality of emitters is varied by altering its intensity.
10. The contactless patient monitor of any one of clauses 7 to 9, wherein the operation of or more of the plurality of emitters is varied by altering its position to reduce shadows in the field of view.
11. A method of monitoring a patient includes controlling the operation of at least one emitter, and at least one detector, the method comprising
   varying the operation of the emitter in response to the environmental conditions in its field of view as detected by the at least one detector to improve an image captured by the at least one detector.
12. The method of clause 11, wherein the detector is a high-resolution camera and the operation of the emitter is varied based on the camera signal.
13. The method of clause 12, including varying the operation of the emitter by altering its wavelength.
14. The method of either clause 12 or clause 13, including varying the operation of the emitter by altering its intensity.
15. The method of any one of clauses 12 to 14, including varying the operation of the emitter by altering its position to reduce shadows in the field of view.
16. The method of clause 12, wherein a plurality of emitters are positioned to emit into a field of view of the high resolution camera and the operation of multiple ones of the plurality of emitters is varied based on the camera signal.
17. The method of clause 16, including varying the operation of at least one emitter by altering its wavelength.
18. The method of either clause 16 or clause 17, including varying the operation of at least one emitter by altering its intensity.
19. The method of any one of clauses 16 to 18, including varying the operation of at least one emitter by altering its position to reduce shadows in the field of view.
20. A contactless monitoring unit for monitoring a patient in a care environment, the contactless monitoring unit comprising:
   a contactless monitor including at least one emitter and first and second detectors,
   a controller including a processor and a non-transitory memory device, the non-transitory memory device including instructions that, when executed by the processor, cause the contactless monitoring unit to acquire information from the environment in which a patient being monitored by the contactless monitoring unit is located and modify operation of the contactless monitor based on the information from the environment.
21. The contactless monitoring unit of clause 20, wherein the first detector is a high resolution monochromatic detector operable, the second detector is a low resolution infrared camera, and wherein the controller varies the operation of the emitter in the contactless monitoring sensor in response to the environmental condition in the field of view of the contactless monitoring sensor as detected by the monochromatic detector in the contactless monitoring sensor.
22. The contactless monitoring system of either clause 20 or clause 21, wherein the contactless monitoring unit is in communication with a patient support apparatus, wherein the field of view as detected by the monochromatic detector in the contactless monitoring sensor includes a patient on the support apparatus, and wherein the field of view as detected by the infrared detector in the contactless monitoring sensor includes a patient on the support apparatus.
23. The contactless monitoring unite of clause 22, wherein the contactless monitoring unit is in further communication with an in room computer.
24. The contactless monitoring unit of any one of clauses 20 to 23, wherein the controller defines an operating boundary for the contactless monitor by processing the input from the field of view of the contactless monitoring sensor as obtained by the monochromatic detector.
25. The contactless monitoring unit of any one of clauses 20 to 24, wherein the controller identifies at least one region of interest as a contour by processing signals from both detectors in tandem.
26. The contactless monitoring unit of clause 25, wherein a first region of interest includes the patient's face, wherein the centroid of the patient's face is located on a thermal image obtained from the second detector and mapped onto a grayscale image obtained from the first detector using geometric transformation.
27. The contactless monitoring unit of either clause 25 or clause 26, wherein any changes in the thermal image are updated on the grayscale image, altering the region of interest in real time.
28. The contactless monitoring unit of clause 27, wherein the controller obtains vital information about the patient's health by processing the pixel values obtained from the region of interest in the grayscale image.
29. The contactless monitoring unit of clause 28, wherein the controller obtains raw data from the second detector and converts it into a pseudo colored thermal image by normalizing it and applying a color-map.
30. The contactless monitoring unit of clause 29, wherein the controller identifies contours in upper and lower zones of the patient support apparatus.
31. The contactless monitoring unit of clause 30, wherein the controller chooses a contour based on hard coded criteria and contour location.
32. The contactless monitoring unit of clause 31, wherein the coordinates of the chosen contour is transformed into the coordinates obtained from the monochromatic detector by scaling it by a factor of the monochromatic detector's resolution divided by the infrared detector's resolution and shifting each point by X and Y offsets determined by the common pixels within their intersecting fields of view of both detectors.
33. The contactless monitoring unit of clause 32, wherein the controller obtains vital information about the patient's health by processing the pixel values obtained from the contours in the grayscale image.

## Claims

1. A contactless patient monitor comprising:
at least one emitter,
a first detector, and
a second detector,
a controller controlling the monitor, the controller including a processor and a non-transitory memory device that includes instructions that are performed by the processor to control the monitor, and
wherein the controller varies the operation of the emitter in response to the environmental conditions in its field of view as detected by the first detector to improve the image captured by the first detector from the field of view.

2. The contactless patient monitor of claim 1, wherein the first detector is a high-resolution monochromatic detector operable to detect in its spectrum, and wherein the second detector is a low-resolution thermal camera.

3. The contactless patient monitor of either claim 1 or claim 2, wherein the emitter has a variable output.

4. The contactless patient monitor of claim 3, wherein the operation of the emitter is varied by altering its wavelength and/or by altering its intensity and/or by altering its position to reduce shadows in the field of view.

5. A method of monitoring a patient includes controlling the operation of at least one emitter, and at least one detector, the method comprising
varying the operation of the emitter in response to the environmental conditions in its field of view as detected by the at least one detector to improve an image captured by the at least one detector.

6. The method of claim 5, wherein the detector is a high-resolution camera and the operation of the emitter is varied based on the camera signal.

7. The method of claim 6, including varying the operation of the emitter by altering its wavelength and/or by altering its intensity and/or by altering its position to reduce shadows in the field of view.

8. A contactless monitoring unit for monitoring a patient in a care environment, the contactless monitoring unit comprising:
a contactless monitor as claimed in any one of claims 1 to 4;
the non-transitory memory device including instructions that, when executed by the processor, cause the contactless monitoring unit to acquire information from the environment in which a patient being monitored by the contactless monitoring unit is located and modify operation of the contactless monitor based on the information from the environment.

9. The contactless monitoring unit of claim 8, wherein the controller varies the operation of the emitter in the contactless monitoring sensor in response to the environmental condition in the field of view of the contactless monitoring sensor as detected by the first detector in the contactless monitoring sensor.

10. The contactless monitoring system of claim 9, wherein the contactless monitoring unit is in communication with a patient support apparatus, wherein the field of view as detected by the first detector in the contactless monitoring sensor includes a patient on the support apparatus, and wherein the field of view as detected by the second detector in the contactless monitoring sensor includes the patient on the support apparatus.

11. The contactless monitoring unit of either claim 9 or claim 10, wherein the controller defines an operating boundary for the contactless monitor by processing the input from the field of view of the contactless monitoring sensor as obtained by the first detector.

12. The contactless monitoring unit of any one of claims 8 to 11, wherein the controller identifies at least one region of interest as a contour by processing signals from both detectors in tandem.

13. The contactless monitoring unit of claim 12, wherein a first region of interest includes the patient's face, wherein the centroid of the patient's face is located on a thermal image obtained from the second detector and mapped onto a grayscale image obtained from the first detector using geometric transformation.

14. The contactless monitoring unit of claim 13, wherein the controller obtains vital information about the patient's health by processing the pixel values obtained from the region of interest in the grayscale image.

15. The contactless monitoring unit of claim 14, wherein the controller obtains raw data from the second detector and converts it into a pseudo colored thermal image by normalizing it and applying a color-map.
